# EUROPÄISCHE PATENTSCHRIFT

(11) **EP 1 198 204 B1**
(45) Veröffentlichungstag und Bekanntmachung des Hinweises auf die Patenterteilung: **05.02.2003**
(21) Anmeldenummer: 00953078.3
(22) Anmeldetag: 21.07.2000
(51) Int. Cl.: A61B 17/128

(54) **ANLEGEINSTRUMENT FÜR CHIRURGISCHE CLIPS**
INSTRUMENT FOR PLACING SURGICAL CLIPS
INSTRUMENT POUR POSER DES CLIPS CHIRURGICAUX

(30) Priorität: 23.07.1999 DE 19934634
(43) Veröffentlichungstag der Anmeldung: 24.04.2002
(73) Patentinhaber: Aesculap AG & Co. KG, 78532 Tuttlingen (DE)
(72) Erfinder: MAYENBERGER, Rupert, D-78239 Rielasingen (DE)
(74) Vertreter: Böhme, Ulrich, Dr. Dipl.-Phys.
(86) Internationale Anmeldenummer: EP0007010
(87) Internationale Veröffentlichungsnummer: WO01006936

(56) Entgegenhaltungen:
- WO-A-99/27859
- DE-A- 19 603 889
- US-A- 4 430 997
- US-A- 4 512 345

## Beschreibung

Die Erfindung betrifft ein Anlegeinstrument für U-förmige chirurgische Clips mit einem ein distales und ein proximales Ende aufweisenden Magazin, in dem mehrere Clips hintereinander in einer Führung längsverschieblich gelagert sind, mit einer Vorschubplatte an der Unterseite des Magazins, die mittels federnder Laschen an der Rückseite der Clips anliegt und die mittels einer Vorschubeinrichtung über mindestens einen Clipabstand parallel zur Führung des Magazins vor- und zurückschiebbar ist und dabei bei der Vorschubbewegung die Clips gemeinsam in Richtung auf das distale Ende des Magazins verschiebt, wobei die Vorschubeinrichtung an der Vorschubplatte beim Vorschieben derselben an einem Punkt angreift, der vom proximalen Ende der Vorschubplatte um mindestens die halbe Länge der Vorschubplatte entfernt ist.

Ein Anlegeinstrument für U-förmige chirurgische Clips ist beispielsweise aus der DE 196 03 889 A1 bekannt. Bei diesem bekannten Anlegeinstrument greift eine stangenförmige Vorschubeinrichtung am proximalen Ende der Vorschubplatte an dieser an und schiebt diese bei der Vorschubbewegung nach vorne und zieht sie wieder zurück. Bei der Vorschubbewegung muß dabei die Vorschubplatte Schubkräfte auf alle im Magazin angeordneten Clips übertragen, die bei dieser Vorschubbewegung jeweils um eine Position nach vorne verschoben werden.

Bei herkömmlichen Anlegeinstrumenten ist dies ohne weiteres möglich, es ergeben sich aber erhebliche Schwierigkeiten dann, wenn das Anlegeinstrument verkleinert werden soll, wenn also insbesondere der Außendurchmesser des Instrumentes verkleinert werden soll. Bei einer Reduzierung dieses Durchmessers müssen die im Schaft angeordneten Bauteile ebenfalls in ihren Abmessungen reduziert werden, und hier können sich Festigkeitsprobleme bei den einzelnen Teilen ergeben.

In der US-A-4,430,997, auf der die zweiteilige Form des Anspruchs 1 basiert, ist ein Clipanlegegerät der eingangs genannten Art beschrieben, bei dem die genannten Schwierigkeiten dadurch überwunden werden, daß die Vorschubeinrichtung an der Vorschubplatte in deren Mitte angreift. Durch eine solche Ausgestaltung wird verhindert, daß die Vorschubplatte beim Vorschieben vom proximalen Ende aus vorgeschoben wird, es wird also die halbe Länge der Vorschubplatte gezogen und nur die halbe Länge geschoben. Die Beanspruchung auf Zug ist bei der Vorschubbewegung für die Vorschubplatte günstiger, da ein Ausknicken der Vorschubplatte unter der Kraft von Schubkräften vermieden wird.

Allerdings reicht diese Maßnahme insbesondere bei sehr kleinen Dimensionen und damit bei der Verwendung von sehr dünnen Materialien für die Vorschubplatten nicht aus, um mit Sicherheit ein Ausknicken der Vorschubplatte zu verhindern.

Es ist daher Aufgabe der Erfindung, ein Anlegeinstrument der gattungsgemäßen Art so weiterzubilden, daß auch bei einer Verkleinerung der Abmessungen ohne weiteres die hohen Kräfte zur Verfügung gestellt werden können, die zum Vorschieben der Clips notwendig sind, ohne daß eine Beschädigung der Vorschubplatte erfolgt.

Diese Aufgabe wird bei einem Anlegeinstrument der eingangs beschriebenen Art erfindungsgemäß dadurch gelöst, daß die Vorschubplatte in ihrem distal des Angriffspunktes der Vorschubeinrichtung angeordneten Bereich einen nach unten vorstehenden Mittelbereich aufweist, der im Abstand von den im Magazin geführten Clips verläuft, und daß die Vorschubplatte in ihrem proximal des Angriffspunktes der Vorschubeinrichtung angeordneten Bereich einen nach oben vorstehenden Mittelbereich aufweist, der dicht an den im Magazin geführten Clips verläuft. Der nach unten vorstehende Mittelbereich bildet dabei eine Versteifungssicke aus, die diesen Bereich der Vorschubplatte gegen unerwünschtes Ausknicken verstärkt, so daß in diesem Bereich, der maximal die halbe Länge der Vorschubplatte einnimmt, relativ hohe Schubkräfte übertragen werden können. Auch der proximal des Angriffspunktes der Vorschubeinrichtung angeordnete Bereich kann nach Art einer Sicke ausgebildet sein und dient der Versteifung, außerdem wird dadurch im proximalen Bereich der Vorschubplatte unterhalb derselben ein Raum zur Aufnahme der Vorschubeinrichtung geschaffen, so daß dadurch insgesamt die Bauhöhe des Anlegeinstrumentes reduzierbar ist. Günstig ist es dabei auch, daß durch die unterschiedliche Richtung der Sicken im distalen Bereich und im proximalen Bereich der Vorschubplatte eine zusätzliche Versteifung erreicht werden kann.

Gemäß einer besonders bevorzugten Ausführungsform ist vorgesehen, daß die Vorschubeinrichtung ein unter der Vorschubplatte angeordneter, vor- und zurückschiebbarer Stab ist, der beim Vorschieben mit einem seitlichen Vorsprung an einer Kante der Vorschubplatte zur Anlage kommt, die nach unten aus der Ebene der Vorschubplatte vorsteht.

Vorzugsweise wird diese Kante durch den nach unten vorstehenden Mittelbereich der Vorschubplatte gebildet. Dadurch ist es im Gegensatz zu bekannten Anlegeinstrumenten nicht mehr notwendig, die Vorschubeinrichtung an einer nach unten gerichteten, elastischen Zunge der Vorschubplatte anliegen zu lassen, die bei höherer Beanspruchung unerwünscht verformt werden könnte, sondern die Krafteinleitung erfolgt durch den nach unten vorstehenden Mittelbereich der Vorschubplatte, so daß die Vorschubeinrichtung kein Drehmoment auf ein mit der Vorschubplatte verbundenes Teil ausübt, sondern direkt in Vorschubrichtung die Vorschubkräfte in die Vorschubplatte einleiten kann.

Bei einer weiteren bevorzugten Ausführungsform ist vorgesehen, daß der Stab proximal des seitlichen Vorsprungs dicht an dem Mittelbereich der Vorschubplatte angeordnet ist und Rücksprünge aufweist, die beim Zurückschieben des Stabes Aufnahmeräume für die federnden Laschen der Vorschubplatte bilden.

Eine solche Ausgestaltung ermöglicht es einerseits, die Vorschubplatte im proximalen Bereich sehr dicht an den Clips anzuordnen und außerdem den Raum unmittelbar unterhalb der Vorschubplatte für die Vorschubeinrichtung zu nutzen. Trotz dieser sehr kompakten Anordnung können die an den Clips anliegenden federnden Laschen beim Zurückziehen der Vorschubplatte an der Unterseite der Clips vorbeigleiten, da sie in die Freiräume der Vorschubeinrichtung eintauchen können und somit genügend Platz haben, um den an ihnen vorbeigleitenden Clips auszuweichen. Insgesamt erhält man dadurch eine besonders kompakte Anordnung.

Günstig ist es, wenn der Stab einen kreisförmigen Querschnitt hat und wenn der seitliche Vorsprung und die Rücksprünge durch umlaufende Ringschultern bzw. Ringnuten gebildet werden. Bei einer solchen Ausgestaltung taucht die Kante des nach unten vorstehenden Mittelbereiches der Vorschubplatte im distalen Bereich der Vorschubplatte in den Querschnitt des kreisförmigen Stabes ein, so daß der Stab die Vorschubkraft stirnseitig unmittelbar in die Vorschubplatte einleiten kann. Auch bei sehr dünner Ausgestaltung der Vorschubplatte ergibt sich somit eine sichere Krafteinleitung ohne die Gefahr eines seitlichen Ausknickens derselben.

Es kann weiterhin vorgesehen sein, daß die Vorschubeinrichtung beim Zurückschieben mit einem seitlichen Vorsprung an einer federnden Lasche der Vorschubplatte anliegt, wobei die federnde Lasche vorzugsweise am proximalen Ende der Vorschubplatte angeordnet ist, die Vorschubplatte wird also beim Zurückschieben gezogen.

Es ist dabei günstig, wenn die seitlichen Vorsprünge an der Vorschubeinrichtung weniger weit voneinander entfernt sind als die korrespondierenden Angriffspunkte an der Vorschubplatte. Dadurch ist es möglich, die Vorschubeinrichtung mit einer reziprozierenden Bewegung zu versehen, die größer ist als die Schrittweite der im Magazin gelagerten Clips, trotzdem wird die Vorschubplatte nur um jeweils eine Schrittweite der Clips vorund zurückgeschoben. Dies ist besonders dann günstig, wenn bei dem Anlegeinstrument unterschiedliche Handgriffe für die Betätigung der Vorschubeinrichtung verwendet werden sollen, die entsprechend ihrer Bauart unterschiedliche Vorschubbewegungen für die Vorschubeinrichtung erzeugen.

Die Vorschubplatte kann grundsätzlich Teil des Anlegeinstrumentes sein, es ist aber besonders vorteilhaft, wenn die Vorschubplatte parallel zur Führung der Clips längsverschieblich im Magazin geführt ist, also Teil des Magazins ist. Beim Einsetzen des Magazins, welches vorzugsweise seitlich in einen Schaft des Anlegeinstrumentes eingesetzt wird, wird dieses so positioniert, daß die seitlichen Vorsprünge an der Vorschubeinrichtung zwischen die entsprechenden Angriffspunkte an der Vorschubplatte eingreifen, so daß beim Vorschieben und beim Zurückziehen der Vorschubeinrichtung zwangsläufig eine Mitnahme der Vorschubplatte eintritt.

Die Clips können in der Führung des Magazins vorzugsweise in einem lösbaren Klemmsitz gehalten werden, so daß die Clips zwar durch die Vorschubplatte bei deren Vorschubbewegung mitgenommen und um eine Schrittweite nach vorne verschoben werden, jedoch in dieser Position verbleiben, wenn die Vorschubplatte zurückgezogen wird und wenn dabei die an den Clips anliegenden federnden Laschen an den Clips elastisch entlanggleiten und hinter den jeweiligen Clips wieder in die Mitnahmeposition ausfedern.

Die nachfolgende Beschreibung bevorzugter Ausführungsformen der Erfindung dient im Zusammenhang mit der Zeichnung der näheren Erläuterung. Es zeigen:

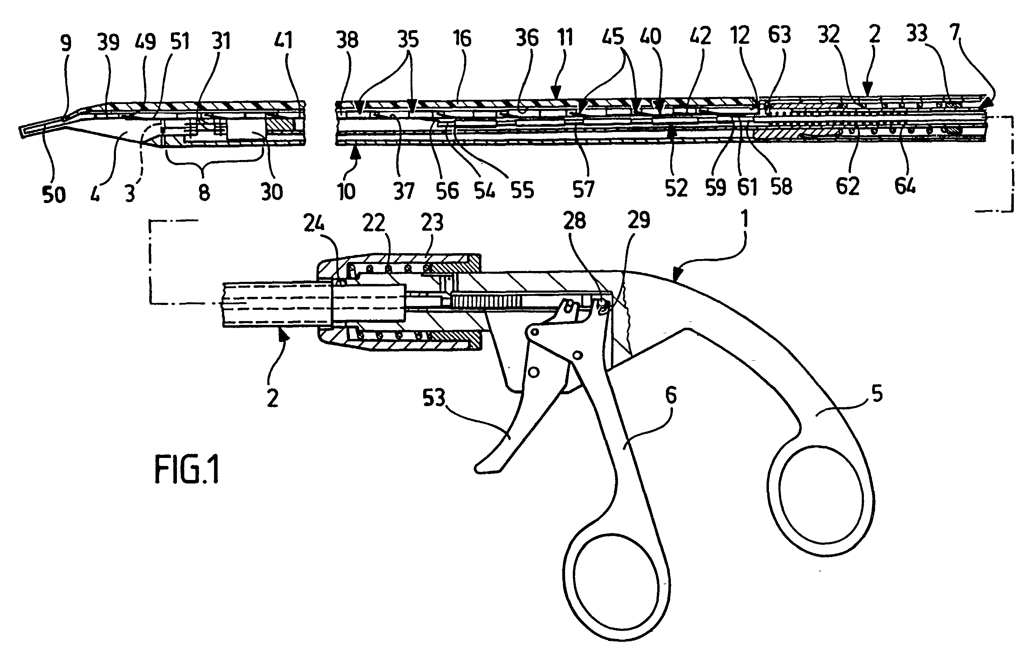

- Figur 1:: eine Längsschnittansicht eines Anlegeinstrumentes für chirurgische Clips;
- Figur 2:: eine perspektivische Explosionsansicht des Schaftteiles des Instrumentes der Figur 1;

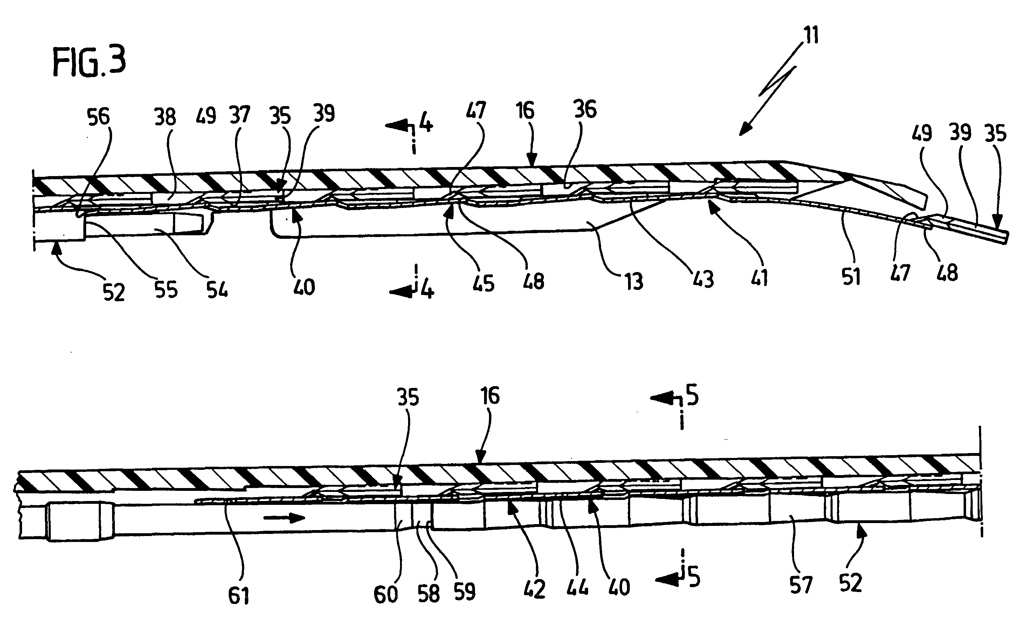

- Figur 3:: eine Längsschnittansicht des Magazins und des unterhalb des Magazins angeordneten Vorschubstabes bei der Vorschubbewegung des Vorschubstabes in distaler Richtung;
- Figur 4:: eine Schnittansicht längs Linie 4-4 in Figur 3;
- Figur 5:: eine Schnittansicht längs Linie 5-5 in Figur 3 und
- Figur 6:: eine Ansicht ähnlich Figur 3 bei der Rückzugbewegung des Vorschubstabes in proximaler Richtung.

Das in der Zeichnung dargestellte Anlegegerät umfaßt einen Griff 1 und einen um seine Längsachse verdrehbar und lösbar an diesem gehaltenen Rohrschaft 2, dessen Außendurchmesser gegenüber seiner Länge gering ist, beispielsweise kann die Länge 25 cm betragen, der Aussendurchmesser 10 mm oder weniger. Am freien Ende des Rohrschaftes 2 sind um eine gemeinsame Schwenkachse 3 verschwenkbar zwei Backen 4 eines zangenförmigen Anlegewerkzeuges gelagert, die über einen im Inneren des Rohrschafts angeordneten Schließmechanismus vom Griff 1 aus geöffnet und geschlossen werden können. Zu diesem Zweck ist der Griff 1 starr mit einer ersten Branche 5 verbunden, gegenüber welcher eine zweite Branche 6 verschwenkbar am Griff 1 gelagert ist. Diese zweite Branche 6 ist gelenkig mit einer Schub- und Zugstange in Form einer Hülse 7 verbunden, die sich durch den gesamten Rohrschaft 2 bis zu dem Schließmechanismus 8 erstreckt. Durch Verschwenken der Branchen 5 und 6 gegeneinander können also die Backen 4 des Anlegewerkzeuges geöffnet und geschlossen werden, wie dies bei Rohrschaftinstrumenten bekannt ist.

Unmittelbar anschließend an das durch die Backen 4 gebildete Anlegewerkzeug weist der Rohrschaft 2 eine sich über den vorderen Teil des Rohrschaftes erstreckende Durchbrechung 9 auf, die sich in Umfangsrichtung über etwa 180° erstreckt, so daß der Rohrschaft 2 im Bereich dieser Durchbrechung nur in der unteren Hälfte des Rohrschaftes durch eine untere Schale 10 abgeschlossen ist, auf der Oberseite jedoch offen.

Die Durchbrechung 9 auf der Oberseite des Rohrschaftes 2 kann geschlossen werden durch ein in diese Durchbrechung 9 eingesetztes Clipmagazin 11, welches im wesentlichen einen fast halbkreisförmigen Querschnitt aufweist und welches den Querschnitt des Rohrschaftes 2 zu einem Vollkreis ergänzt. Dieses Clipmagazin 11 weist an seiner griffnahen proximalen Seite einen Vorsprung 12 auf, der unmittelbar angrenzend an die Durchbrechung 9 geringfügig in den Rohrschaft 2 eintaucht und dadurch die die Durchbrechung 9 begrenzende Kante untergreift. Am vorderen Ende sind seitlich an das Clipmagazin 11 zwei seitliche Lappen 13 angeformt, die in komplementäre Ausnehmungen der unteren Schale 10 eingreifen und in zusammengesetztem Zustand mit nach innen vorspringenden Endbereichen in entsprechende in der Zeichnung nicht dargestellte Nuten der unteren Schale 10 einschnappen, d. h. in diesem Bereich ergibt sich eine elastische Verrastung der Lappen 13 mit den Seitenwänden der unteren Schale 10. Durch das Einstecken des Vorsprunges 12 in den Rohrschaft 2 und durch diese Rastverbindung wird das Clipmagazin 11 stabil am Rohrschaft 2 festgelegt, diese Verbindung ist jedoch wieder lösbar, da die Schnappverbindung durch kräftiges Abziehen des Clipmagazins von der unteren Schale 10 wieder gelöst werden kann.

Der Rohrschaft 2 taucht in eine am Griff 1 in Längsrichtung gegen die Wirkung einer Feder 22 verschiebbare Griffhülse 23 ein, die Teil eines den Rohrschaft 2 mit dem Griff 1 verbindenden Kugelgesperres ist. Diese auf dem Griff 1 in Längsrichtung verschieblich gelagerte Griffhülse 23 legt mehrere im Griff in Radialöffnungen verschiebbare kugelförmige Verriegelungskörper 24 in einer radial innenliegenden Stellung fest, wenn die Feder 22 entspannt ist, sie ermöglicht jedoch eine radiale Auswärtsbewegung dieses Verriegelungskörpers 24, wenn die Griffhülse 23 gegen die Wirkung der Feder 22 zurückgezogen wird. In der radial eingeschobenen Stellung tauchen die Verriegelungskörper 24 in radiale Ausnehmungen des Rohrschaftes ein und legen diesen in axialer Richtung am Griff fest, in ihrer radial nach außen verschobenen Stellung geben die Verriegelungskörper 24 aber den Rohrschaft 2 frei, so daß er in axialer Richtung aus dem Griff 1 herausgezogen werden kann.

Die im Rohrschaft 2 angeordnete Hülse 7 tritt durch die Verbindungsstelle zwischen Rohrschaft 2 und Griff 1 hindurch bis in den Griff ein und ist dort über zwei seitliche Zapfen 28, die in seitliche Öffnungen 29 eingreifen, gelenkig mit der Branche 6 verbunden. Beim Verschwenken der Branche 6 gegenüber der Branche 5 wird die Hülse 7 dadurch in eine distale Stellung vor und in eine proximale Stellung zurückgeschoben.

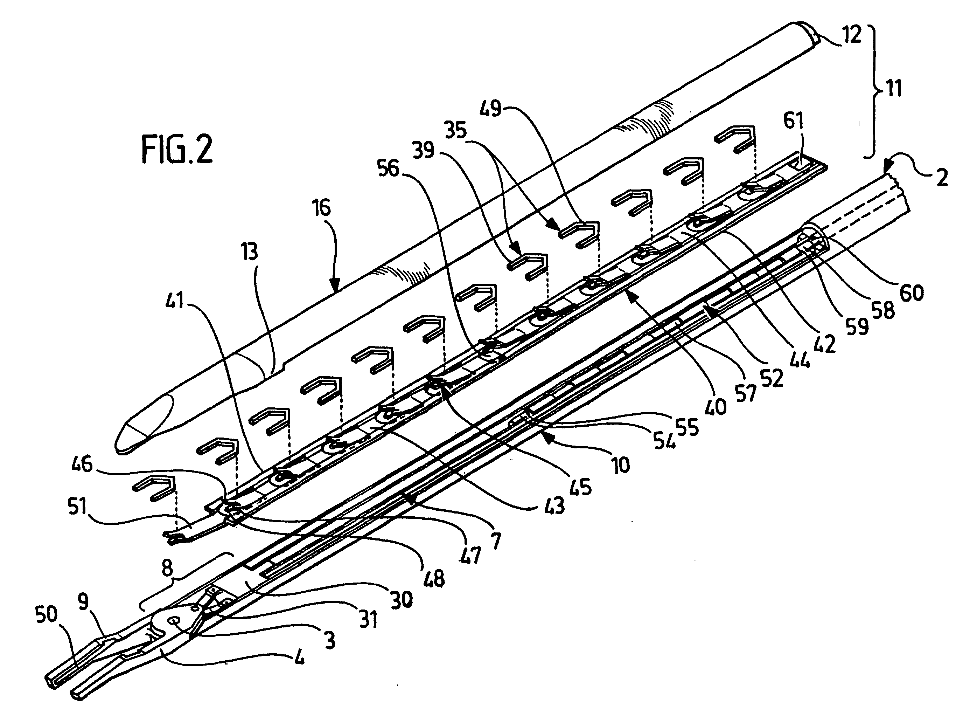

Sie ist im Bereich der Durchbrechung 9 des Rohrschaftes 2 nach oben hin aufgeschnitten, so daß sie in diesem Bereich nur eine untere Schale ausbildet, die an ihrem freien Ende fest mit einem Gleitstein 30 verbunden ist, der den Querschnitt der unteren Schale 10 ausfüllend in dieser längsverschieblich gelagert ist. Dieser Gleitstein 30 ist über jeweils einen Verbindungshebel 31 gelenkig mit einer Backe 4 des Anlegewerkzeugs verbunden, so daß jeweils eine Backe 4 und ein Verbindungshebel 31 einen Kniehebel ausbilden (Figur 2). Dieser Kniehebel kann durch Vor- und Zurückschieben des Gleitsteins 30 mehr oder weniger stark eingeknickt werden, wobei eine Verschwenkung der Backen 4 eintritt. Beim Zurückziehen des Gleitsteins 30 erfolgt wieder eine Öffnung der Bakken 4. Diese Öffnung wird im übrigen unterstützt durch eine Schraubenfeder 32, die die Hülse 7 koaxial umgibt und sich mit einem Ende an einem Vorsprung der unteren Schale 10 und am anderen Ende an einer Ringschulter 33 abstützt, die dauerhaft mit der Hülse 7 verbunden ist. Dadurch wird die Hülse 7 in Richtung auf den Griff 1 mit einer Federkraft beaufschlagt, die die Öffnung der Backen 4 unterstützt.

Der den Gleitstein 30, die Verbindungshebel 31 und den rückwärtigen Teil der Backen 4 umfassende Schließmechanismus 8 ist vollständig in der unteren Schale 10 untergebracht und läßt somit die gesamte Durchbrechung 9 frei zur Aufnahme eines Vorschubmechanismus für die Clips 35 und zur Aufnahme des Clipmagazins 11, in dem sich die Clips 35 befinden.

In dem Clipmagazin 11 ist eine nach unten hin offene Aufnahmekammer 36 für die Clips 35 angeordnet, die die Form einer Längsnut im Clipmagazin 11 aufweist. In die Seitenwände dieser Aufnahmekammer 36 sind einander gegenüberliegend Führungsnuten 38 eingelassen, in welche die Schenkel 39 der U-förmigen Clips 35 eintauchen. Mehrere dieser U-förmigen Clips 35 sind auf diese Weise hintereinander in der Aufnahmekammer 36 angeordnet, wobei sie relativ zueinander einen Abstand einhalten (Figur 2).

Parallel zu den Führungsnuten 38 verläuft in den Seitenwänden jeweils eine weitere Führungsnut 37, und in diesen ist längsverschieblich eine sich über die gesamte Länge des Clipmagazins 11 erstreckende, flächige Vorschubplatte 40 gehalten, die die Aufnahmekammer 36 nach unten hin abschließt. Diese Vorschubplatte 40 weist einen den Backen 4 benachbarten distalen Abschnitt 41 und einen dem Griff 1 zugewandten proximalen Abschnitt 42 auf, die im dargestellten Ausführungsbeispiel etwa gleich lang ausgebildet sind, sich also jeweils über die Hälfte der Gesamtlänge der Vorschubplatte 40 erstrecken.

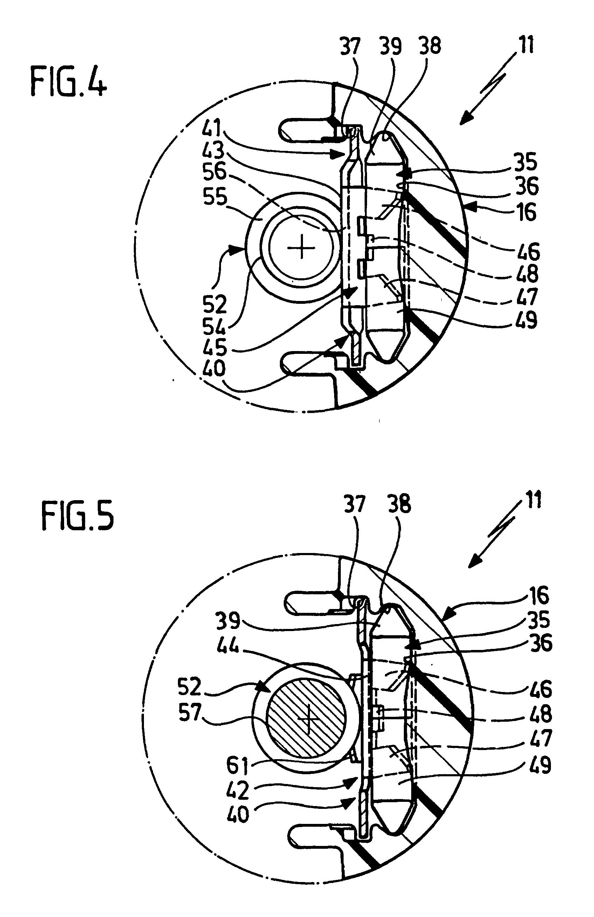

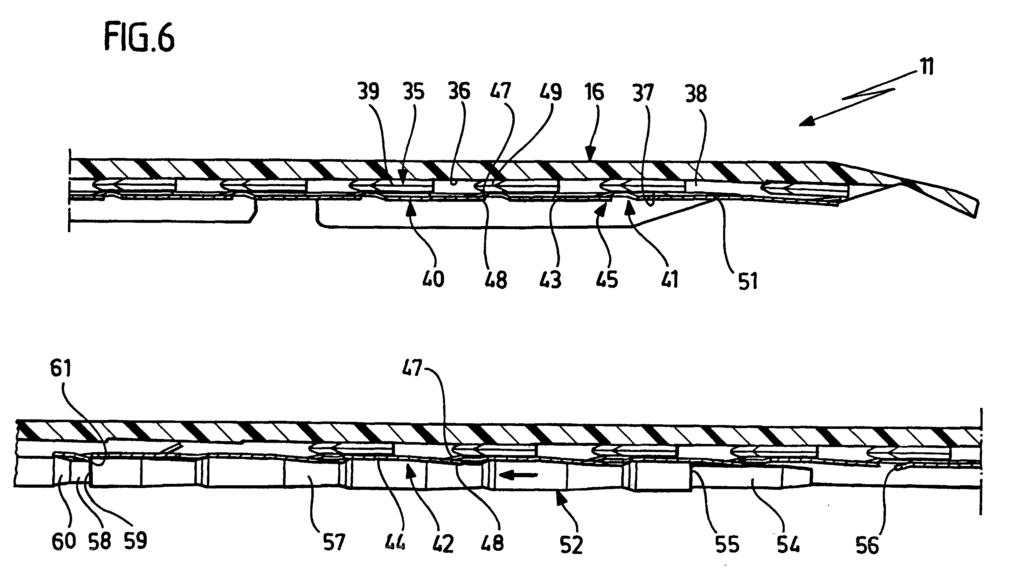

Im distalen Bereich 41 ist der Mittelbereich 43 der Vorschubplatte 40 nach Art einer Verstärkungssicke nach unten verformt, so daß die durch den ebenen Mittelbereich 43 aufgespannte Ebene gegenüber der Ebene versetzt ist, die durch die Führungsnuten 37 aufgespannt wird (Figur 4). Im proximalen Abschnitt 42 ist dagegen der entsprechende Mittelbereich 44 nach oben verformt, d. h. in Richtung auf die Clips 35, so daß der ebene Mittelbereich 44 gegenüber der Ebene der Führungsnuten 37 nach oben versetzt ist (Figur 5). Dadurch liegt der proximale Mittelbereich 44 unmittelbar an der Unterseite der Clips 35 an, der distale Mittelbereich 43 hingegen hält von der Unterseite der Clips 35 einen Abstand ein.

Sowohl im distalen Mittelbereich 43 als auch im proximalen Mittelbereich 44 sind durch Längsschnitte mehrere federnde Laschen 45 ausgebildet, die jeweils an ihrem vorderen Ende durch Längsschnitte in drei nebeneinander angeordnete Federzungen 46, 47, 48 aufgeteilt sind. Die beiden äußeren Federzungen 46, 48 sind im Bereich ihres freien Endes zur Aufnahmekammer 36 hin schräg abgebogen, so daß die freien Kanten zur Fläche der Vorschubplatte 40 schräg verlaufen und über diese Fläche nach oben zur Aufnahmekammer 36 hin vorstehen.

Wenn die Vorschubplatte 40 sich in einer zurückgezogenen, proximalen Position befindet, liegen diese Kanten jeweils an der die beiden Schenkel 39 verbindenden Brücke 49 eines Clips 35 an, wobei die schräg verlaufenden Kanten sicherstellen, daß auch bei einer linienförmigen Kontur der Brücke 49 eine sichere Anlage erfolgt und ein Abgleiten der Kanten vermieden wird. Die mittlere Federzunge 47 der federnden Lasche 45 legt sich an die Unterseite der Clips 35 an, so daß auf diese Weise auch sicher vermieden wird, daß die Kanten an der Oberseite über die Clips 35 hinweggleiten.

Wenn die Vorschubplatte 40 in distaler Richtung verschoben wird, nimmt somit jedes Paar von Federzungen 46, 48 einen Clip 35 mit und schiebt diesen im Clipmagazin 11 um die Schrittweite der Verschiebung der Vorschubplatte 40 in distaler Richtung.

Der vorderste Clip 35 wird dabei aus den Führungsnuten 38 in den Seitenwänden der Aufnahmekammer 36 nach vorne ausgeschoben und gelangt in mit den Führungsnuten 38 ausgerichtete, sich daran anschließende Führungsnuten 50 an den Innenseiten der Backen 4.

An seinem vorderen Ende läuft die Vorschubplatte in einer Federzunge 51 aus, die insgesamt schmaler ausgebildet ist als die Vorschubplatte 40. Beim Vorschieben der Vorschubplatte 40 tritt diese Federzunge 51 zwischen die Backen 4 ein und schiebt den vordersten Clip 35 in die etwas nach unten geneigten Führungsnuten 50 der Backen 4 bis an deren vorderes Ende. Durch die schmale federnde Ausführung der Federzunge 51 im vorderen Teil kann diese ohne weiteres der etwas geänderten Neigung der Führungsnuten 50 folgen.

Das Clipmagazin 11 besteht im wesentlichen aus einer oberen, deckelähnlichen Halbschale 16, in die die Clips 35 und die Vorschubplatte 40 in der beschriebenen Weise eingesetzt sind. In Figur 2 sind diese Teile zur besseren Übersichtlichkeit getrennt voneinander dargestellt.

Bei der Rückbewegung der Vorschubplatte 40 gleiten die federnden Laschen 45 an den nachfolgenden Clips 35 entlang, bis sie wieder vollständig hinter diesen stehen und wieder in die Mitnahmeposition nach oben federn können, in der die Kanten an den Brücken 49 der Clips 35 anliegen. Allein durch die Vor- und Rückbewegung der Vorschubplatte 40 wird jeweils ein Clip zwischen die Backen 4 eingeschoben, der dann in der beschriebenen Weise durch Schließen der Backen angelegt werden kann, beispielsweise zum Verschließen eines Blutgefäßes. Dies erfolgt, bis das Clipmagazin 11 geleert ist, anschließend kann in einfacher Weise ein neues Clipmagazin anstelle des geleerten eingesetzt und die Tätigkeit fortgesetzt werden.

Die Verschiebung der Vorschubplatte 40 erfolgt durch eine Schub- und Zugstange 52, die im Innern der Hülse 7 längsverschieblich geführt ist und die gelenkig mit einem Schwenkhebel 53 am Griff 1 verbunden ist. Dessen Schwenkbewegung führt dazu, daß die Schub- und Zugstange 52 im Innern des Rohrschafts und im Innern der Hülse 7 verschoben werden kann.

Die Schub- und Zugstange 52 weist einen kreisförmigen Querschnitt auf und erstreckt sich unterhalb des proximalen Mittelbereiches 44 der Vorschubplatte 40 über den gesamten proximalen Abschnitt 42. Dabei liegt die Schub- und Zugstange 52 mit ihrem Außendurchmesser dicht an dem proximalen Mittelbereich 44 an.

An ihrem freien Ende weist die Schub- und Zugstange 52 einen kurzen Abschnitt 54 mit einem geringeren Außendurchmesser auf, dadurch wird eine umlaufende Stufe 55 zwischen dem Abschnitt 54 und der übrigen Schub- und Zugstange 52 ausgebildet. Diese Stufe 55 legt sich beim Vorschieben der Schub- und Zugstange in distaler Richtung an eine Kante 56 des distalen Mittelbereiches 43 an, diese Kante wird ausgebildet im Übergangsbereich zwischen dem distalen Mittelbereich 43 und dem proximalen Mittelbereich 44, die nach entgegengesetzten Seiten aus der Ebene hervorstehen, die durch die Führungsnuten 37 aufgespannt wird.

In proximaler Richtung schließen sich an die Stufe 55 mehrere umlaufende Ringnuten 57 in der Schub- und Zugstange 52 an, deren Abstand zueinander dem Abstand der Clips 35 im Clipmagazin 11 entspricht. Diese Ringnuten 57 bilden Aufnahmeräume für die federnden Laschen 45 aus, in die diese eintreten können, wenn sie beim Zurückziehen der Vorschubplatte 40 in die proximale Position an der Unterseite der Clips 35 entlanggleiten und dadurch nach unten verbogen werden.

Im Bereich des proximalen Endes des Clipmagazins 11 ist anschließend an die Ringnuten 57 eine weitere Ringnut 58 in die Schub- und Zugstange 52 eingearbeitet, die an ihrem distalen Ende eine Ringstufe 59 ausbildet und die an ihrem proximalen Ende eine Aufgleitfläche 60 aufweist. Beim Zurückziehen der Vorschubplatte 40 in die proximale Stellung rastet eine Federzunge 61 am proximalen Ende des proximalen Mittelbereichs 44 nach unten in diese Ringnut 58 ein und legt sich mit ihrer freien Kante an die Ringstufe 59 an, so daß die Schub- und Zugstange 52 bei ihrer proximalen Verschiebebewegung die Vorschubplatte 40 in proximaler Richtung mitnimmt. Dadurch wird durch das Vorschieben der Schub- und Zugstange 52 und durch deren Zurückschieben jeweils auch die Vorschubplatte 40 vor- und zurückgeschoben. Beim Vorschieben in distaler Richtung erfolgt dabei die Kraftübertragung über die Stufe 55, die sich an die Kante 56 anlegt, beim Zurückziehen in proximaler Richtung dagegen durch die Federzunge 61, die sich an die Ringstufe 59 anlegt.

Der Abstand der Kante 56 einerseits und der Federzunge 61 andererseits ist größer gewählt als der Abstand der Stufe 55 und der Ringstufe 59, so daß die Schub- und Zugstange 52 eine größere Vor- und Rückbewegung ausführen kann und trotzdem die Vorschubplatte 40 dabei in beiden Richtungen nur um einen Clipabstand im Magazin verschiebt. Dadurch ist eine Anpassung an unterschiedliche Vorschubbewegungen verschiedener Griffe möglich.

Die Wirkverbindung zwischen Schub- und Zugstange 52 einerseits und Vorschubplatte 40 andererseits ist lösbar, da sie allein durch die unmittelbare Nachbarschaft der Schub- und Zugstange 52 zu der Vorschubplatte 40 erzeugt wird. Diese Verbindung wird also ohne weiteres hergestellt, wenn ein Clipmagazin 11 in den Rohrschaft 2 eingesetzt wird, sie wird wieder gelöst, wenn das Clipmagazin 11 aus dem Rohrschaft 2 entnommen wird.

Die Schub- und Zugstange 52 wird von einer Schraubenfeder 62 konzentrisch umgeben, die sich einerseits an einem Vorsprung 63 des Rohrschaftes 2 und andererseits an einer Stufe 64 der Schub- und Zugstange 52 abstützt und diese dadurch in die zurückgeschobene, proximale Stellung verschiebt. Ein Vorschieben der Vorschubplatte 40 in distaler Richtung erfolgt somit gegen die Wirkung der Schraubenfeder 62, die sich im übrigen im Innern der Hülse 7 befindet.

## Patentansprüche

1. Anlegeinstrument für U-förmige chirurgische Clips (35) mit einem ein distales und ein proximales Ende aufweisenden Magazin (11), in dem mehrere Clips (35) hintereinander in einer Führung längsverschieblich gelagert sind, mit einer Vorschubplatte (40) an der Unterseite des Magazins (11), die mittels federnder Laschen (45) an der Rückseite der Clips (35) anliegt und die mittels einer Vorschubeinrichtung (52) über mindestens einen Clipabstand parallel zur Führung des Magazins (11) vor- und zurückschiebbar ist und dabei bei der Vorschubbewegung die Clips (35) gemeinsam in Richtung auf das distale Ende des Magazins (11) vorschiebt, wobei die Vorschubeinrichtung (52) an der Vorschubplatte (40) beim Vorschieben derselben an einem Punkt (56) angreift, der vom proximalen Ende der Vorschubplatte (40) um mindestens die halbe Länge der Vorschubplatte (40) entfernt ist, **dadurch gekennzeichnet, daß** die Vorschubplatte (40) in ihrem distal des Angriffspunktes (56) der Vorschubeinrichtung (52) angeordneten Bereich (41) einen nach unten vorstehenden Mittelbereich (43) aufweist, der im Abstand von den im Magazin (11) geführten Clips (35) verläuft, und daß die Vorschubplatte (40) in ihrem proximal des Angriffspunktes (56) der Vorschubeinrichtung (52) angeordneten Bereich (42) einen nach oben vorstehenden Mittelbereich (44) aufweist, der dicht an den im Magazin (11) geführten Clips (35) verläuft.

2. Anlegeinstrument nach Anspruch 1, **dadurch gekennzeichnet, daß** die Vorschubeinrichtung (52) ein unter der Vorschubplatte (50) angeordneter, vor- und zurückziehbarer Stab ist, der beim Vorschieben mit einem seitlichen Vorsprung (55) an der Kante (56) der Vorschubplatte (40) zur Anlage kommt, die nach unten aus der Ebene der Vorschubplatte (40) vorsteht.

3. Anlegeinstrument nach Anspruch 2, **dadurch gekennzeichnet, daß** die Kante (56) gebildet wird durch den nach unten vorstehenden Mittelbereich (43) der Vorschubplatte (40).

4. Anlegeinstrument nach einem der Ansprüche 2 oder 3, **dadurch gekennzeichnet, daß** der Stab (52) proximal des seitlichen Vorsprunges (55) dicht an dem Mittelbereich (44) der Vorschubplatte (40) angeordnet ist und Rücksprünge (57) aufweist, die beim Zurückschieben des Stabes (52) Aufnahmeräume für die federnden Laschen (45) der Vorschubplatte (40) bilden.

5. Anlegeinstrument nach einem der Ansprüche 2 bis 4, **dadurch gekennzeichnet, daß** der Stab (52) einen kreisförmigen Querschnitt hat und daß der seitliche Vorsprung (55) und die Rücksprünge (57) durch umlaufende Ringschultern bzw. Ringnuten gebildet werden.

6. Anlegeinstrument nach einem der voranstehenden Ansprüche, **dadurch gekennzeichnet, daß** die Vorschubeinrichtung (52) beim Zurückschieben mit einem seitlichen Vorsprung (59) an einer federnden Lasche (61) der Vorschubplatte (40) anliegt.

7. Anlegeinstrument nach Anspruch 6, **dadurch gekennzeichnet, daß** die federnde Lasche (61) am proximalen Ende der Vorschubplatte (40) angeordnet ist.

8. Anlegeinstrument nach einem der Ansprüche 6 oder 7, **dadurch gekennzeichnet, daß** die seitlichen Vorsprünge (55; 59) an der Vorschubeinrichtung (52) weniger weit voneinander entfernt sind als die korrespondierenden Angriffspunkte (56; 61) an der Vorschubplatte (40).

9. Anlegeinstrument nach einem der voranstehenden Ansprüche, **dadurch gekennzeichnet, daß** die Vorschubplatte (40) parallel zur Führung (38) der Clips (35) längsverschieblich im Magazin (11) geführt ist.

10. Anlegeinstrument nach einem der voranstehenden Ansprüche, **dadurch gekennzeichnet, daß** die Clips (35) in der Führung (38) des Magazins (11) in einem lösbaren Klemmsitz gehalten sind.

## Claims

1. An instrument for placing U-shaped surgical clips (35), with a magazine (11) having a distal and a proximal end, in which magazine a plurality of clips (35) are positioned one behind the other longitudinally displaceably in a guide, with a forward-feed plate (40) on the underside of the magazine (11), the said forward-feed plate (40) contacting the rear end of the clips (35) by means of elastic tongues (45), being capable - by means of a forward-feed device (52) - of advancement and return over at least one clip distance parallel to the guide of the magazine (11), and also, in association with the forward-feed movement, advancing the clips (35) together in the direction of the distal end of the magazine (11), the forward-feed device (52) engaging with the forward-feed plate (40) during advancement of the same at a point (56) distanced from the proximal end of the forward-feed plate (40) by at least half the length of the forward-feed plate (40), **characterised in that** the forward-feed plate (40) has - in its region (41) positioned distally of the point of engagement (56) of the forward-feed device (52) - a downwardly projecting central region (43) the course of which is distanced from the clips (35) guided in the magazine (11), and **in that** the forward-feed plate (40) has - in its region (42) positioned proximally of the point of engagement (56) of the forward-feed device (52) - an upwardly projecting central region (44) the course of which is close to the clips (35) guided in the magazine (11).

2. A clip-placing instrument according to Claim 1, **characterised in that** the forward-feed device (52) is an advanceable and retractable rod positioned beneath the forward-feed plate (50 [sic, recte 40) which, when advanced, comes to rest with a lateral jutting part (55) against the edge (56) of the forward-feed plate (40) projecting downwards out of the plane of the forward-feed plate (40).

3. A clip-placing instrument according to Claim 2, **characterised in that** the edge (56) is formed by the downwardly-projecting central region (43) of the forward-feed plate (40).

4. A clip-placing instrument according to one of Claims 2 or 3, **characterised in that** the rod (52) is positioned proximally of the lateral jutting part (55) close to the central region (44) of the forward-feed plate (40) and has recesses (57) which, when the rod (52) is returned, form spaces for accommodating the elastic tongues (45) of the forward-feed plate (40).

5. A clip-placing instrument according to one of Claims 2 to 4, **characterised in that** the rod (52) has a circular cross-section and **in that** the lateral jutting part (55) and the recesses (57) are formed by circumferential annular shoulders and annular grooves respectively.

6. A clip-placing instrument according to one of the preceding claims, **characterised in that**, when returned, the forward-feed device (52) lies with a lateral jutting part (59) against an elastic tongue (61) of the forward-feed plate (40).

7. A clip-placing instrument according to Claim 6, **characterised in that** the elastic tongue (61) is positioned at the proximal end of the forward-feed plate (40).

8. A clip-placing instrument according to one of Claims 6 or 7, **characterised in that** the lateral jutting parts (55; 59) on the forward-feed device (52) are less far apart from one another than the corresponding point of engagement (56; 61) on the forward-feed plate (40).

9. A clip-placing instrument according to one of the preceding claims, **characterised in that** the forward-feed plate (40) is guided longitudinally displaceably in the magazine (11) parallel to the guide (38) of the clips (35).

10. A clip-placing instrument according to one of the preceding claims, **characterised in that** the clips (35) are held in a releasable press fit in the guide (38) of the magazine (11).

## Revendications

1. Instrument de pose d'agrafes (35) chirurgicales en forme de U, comprenant un chargeur (11) présentant une extrémité distale et une extrémité proximale, dans lequel plusieurs agrafes (35) sont montées les unes derrière les autres à déplacement longitudinal dans un guidage, comportant une plaque d'avancement (40) sur la face inférieure du chargeur (11), laquelle est en appui sur la face postérieure des agrafes (35) au moyen de languettes (45) élastiques et qui peut être poussée en avant et en arrière au moyen d'un dispositif d'avancement (52) sur au moins un espacement d'agrafe parallèlement au guidage du chargeur (11) et qui, lors du mouvement d'avancement, fait avancer ici ensemble les agrafes (35) en direction de l'extrémité distale du chargeur (11), le dispositif d'avancement (52) attaquant la plaque d'avancement (40) lors de l'avancement de celle-ci, sur un point (56) qui est éloigné de l'extrémité proximale de la plaque d'avancement (40) d'au moins la demi-longueur de la plaque d'avancement (40), **caractérisé en ce que** la plaque d'avancement (40) présente, dans sa région (41) distante du point d'attaque (56) du dispositif d'avancement (52), une région médiane (43) faisant saillie vers le bas qui s'étend à distance des agrafes (35) guidées dans le chargeur (11), et **en ce que** la plaque d'avancement (40) présente, dans sa région proche du point d'attaque (56) du dispositif d'avancement (52), une région médiane (44) faisant saillie vers le haut qui s'étend tout près des agrafes (35) guidées dans le chargeur (11).

2. Instrument de pose selon la revendication 1, **caractérisé en ce que** le dispositif d'avancement (52) est une barre qui peut être poussée en avant et en arrière, agencée au-dessous de la plaque d'avancement (50) et qui, lors de la poussée en avant, vient en appui avec une saillie latérale (55) sur l'arête (56) de la plaque d'avancement (40), arête qui fait saillie vers le bas hors du plan de la plaque d'avancement (40).

3. Instrument de pose selon la revendication 2, **caractérisé en ce que** l'arête (56) est formée par la région médiane (43), en saillie vers le bas, de la plaque d'avancement.

4. Instrument de pose selon l'une ou l'autre des revendications 2 et 3, **caractérisé en ce que** la barre (52) est agencée à proximité de la saillie latérale (55), tout près de la région médiane (44) de la plaque d'avancement (40), et présente des retraits (57) qui, lors de la poussée en arrière de la barre (52), forment des espaces de réception pour les pattes (45) élastiques de la plaque d'avancement (40).

5. Instrument de pose selon l'une des revendications 2 à 4, **caractérisé en ce que** la barre (52) a une section transversale circulaire et **en ce que** la saillie latérale (55) et les retraits (57) sont formés par des épaulements annulaires et des gorges annulaires périphériques, respectivement.

6. Instrument de pose selon l'une des revendications précédentes, **caractérisé en ce que** lors de la poussée vers l'arrière, le dispositif d'avancement (52) est en appui avec une saillie latérale (59) sur une patte élastique (61) de la plaque d'avancement (40).

7. Instrument de pose selon la revendication 6, **caractérisé en ce que** la patte élastique (61) est agencée à l'extrémité proximale de la plaque d'avancement (40).

8. Instrument de pose selon l'une ou l'autre des revendications 6 et 7, **caractérisé en ce que** les saillies latérales (55 ; 59) sur le dispositif d'avancement (52) sont moins éloignées l'une de l'autre que les points d'attaques (56 ; 61) correspondants sur la plaque d'avancement (40).

9. Instrument de pose selon l'une des revendications précédentes, **caractérisé en ce que** la plaque d'avancement (40) est guidée à déplacement longitudinal dans le chargeur (11) parallèlement au guidage (38) des agrafes (35).

10. Instrument de pose selon l'une des revendications précédentes, **caractérisé en ce que** les agrafes (35) sont maintenues en serrage libérable dans le guidage (38) du chargeur (11).
